**Europäisches Patentamt**

**European Patent Office**

**Office européen des brevets**

(11) Publication number : **0 489 695 A2**

## (12) EUROPEAN PATENT APPLICATION

(21) Application number : **91830047.6**

(22) Date of filing : **15.02.91**

(51) Int. Cl.⁵ : **G06F 15/42**

(30) Priority : **04.07.90 IT 2144690 U**

(43) Date of publication of application :
**10.06.92 Bulletin 92/24**

(84) Designated Contracting States :
**AT BE CH DE DK ES FR GB GR LI NL SE**

(71) Applicant : **Da Villa, Giuliano**
**Via dell'Orso, 74**
**Roma (IT)**

(72) Inventor : **Da Villa, Giuliano**
**Via dell'Orso, 74**
**Roma (IT)**

(74) Representative : **Cicogna, Franco**
**Ufficio Internazionale Brevetti Dott.Prof.**
**Franco Cicogna Via Visconti di Modrone, 14/A**
**I-20122 Milano (IT)**

(54) **Monitoring device for monitoring the caloric contents of diets.**

(57) A monitoring device for monitoring the caloric contents of diets comprises a processor controlled by a first digital keyboard and a second keyboard the keys of which bear food symbols related to individual foods or a combination of individual foods, the processor including a fixed store in which there are stored the unit caloric powers of the individual foods and a display on which the unit caloric powers of the foods can be displayed by pressing a related key of the digital keyboard.

FIG. 1

EP 0 489 695 A2

## BACKGROUND OF THE INVENTION

The present invention relates to a monitoring or processing device for monitoring and displaying the caloric contents of diets.

As is known, in the dietetic treatment of several diseases, such as obesity, renal and hepatic insufficiency, iper-cholesteric, iper-triglyceridic diseases, gout, stomach and digestive duct diseases and the like, it is very important to properly know the caloric power of the foods.

However, a calculation of the total calorie amount related to one or more full meals involves rather complex operations, in order to establish the unit caloric power of the individual foods, multiply this individual caloric power by the weight of the foods, and register the result for carrying sum operations and the like.

## SUMMARY OF THE INVENTION

Accordingly, the aim of the present invention is to overcome the above mentioned drawback, by providing a monitoring device, controlled by processor means, which affords the possibility of quickly and accurately calculating the calorie contents of a given amount of a food included in a given diet.

Within the scope of the above mentioned aim, a main object of the present invention is to provide such a monitoring device for monitoring the caloric contents of diets which comprises store means suitable to store a plurality of diet data in order to provide the user with the caloric contents of a single meal, as well as of the meals of a day, a week, and so on.

Another object of the present invention is to provide such a diet monitoring device which is very reliable and of simple operation.

According to one aspect of the present invention, the above mentioned aim and objects, as well as yet other objects, which will become more apparent hereinafter, are achieved by a monitoring device for monitoring the caloric contents of diets, characterized in that said device comprises processor means, a first digital keyboard and a second keyboard, the keys of which bear symbols related to single foods, or to a combination of foods, said first and second keyboards controlling said processor means, said processor means including a fixed store suitable to store a unit caloric power of said foods, and display means for displaying said caloric power of said foods as a key of said digital keyboard is pressed.

## DESCRIPTION OF THE DRAWINGS

Further characteristics and advantages of the monitoring device for monitoring the caloric contents of diets according to the present invention will become more apparent from the following detailed disclosure of a preferred embodiment thereof, which is illustrated, by way of an indicative but not limitative example, in the figures of the accompanying drawings where:

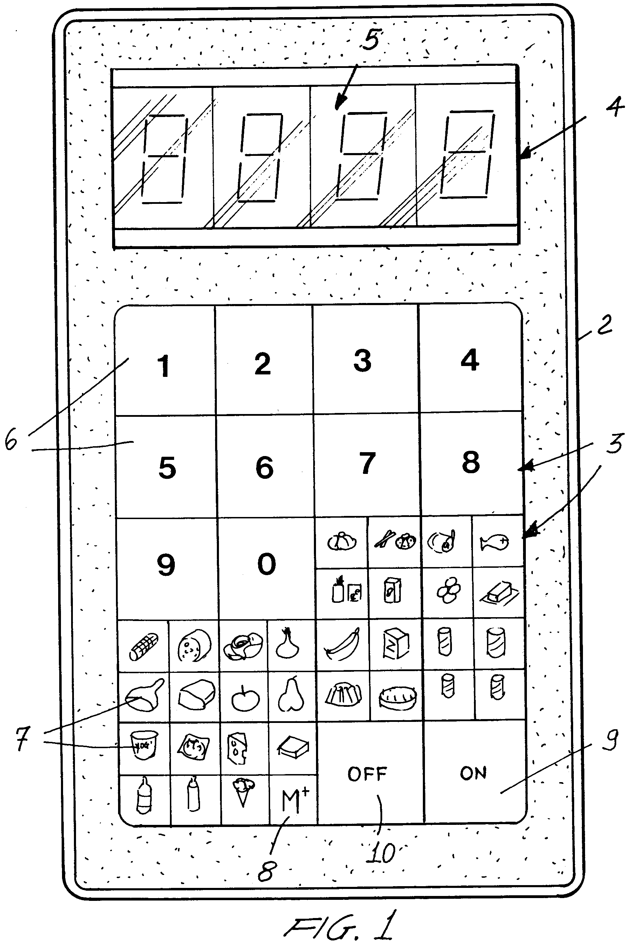

Figure 1 is a schematic top view of the monitoring device according to the present invention; and

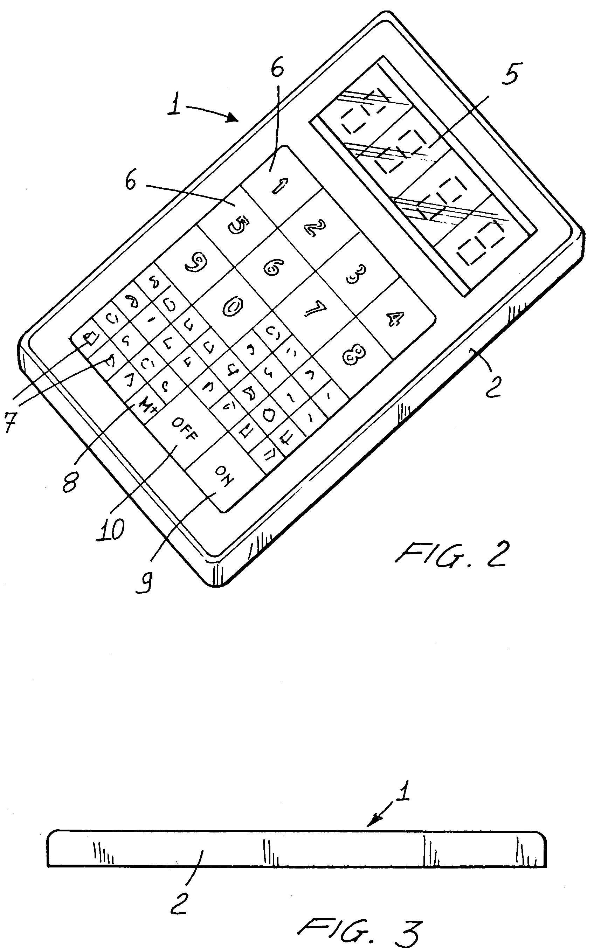

Figures 2 and 3 are respectively a perspective view and a side view of the monitoring device according to the invention.

## DESCRIPTION OF THE PREFERRED EMBODIMENT

With reference to the figures of the accompanying drawings, the monitoring device for monitoring the caloric contents of diets according to the invention, which has been indicated overally at the reference number 1, comprises a processor system, of a conventionally known type, enclosed in a box-like housing 2 including a plurality of windows 3 housing corresponding keys and a cross top opening 4 for holding display means 5, preferably of the liquid crystal type.

More specifically, a portion of the above mentioned keys defines a first digital keyboard 6, by means of which there is set the value of the weight amount of a food to be eaten.

A second portion of the keys 7 bears corresponding figures or symbols indicative of the individual foods, or of combinations of foods, the unit caloric power of which has been preliminarily stored into the store of the mentioned processor means.

The device further comprises a store key 8 as well as an on switch key 9 and an off switch key 10.

The main feature of the present invention is that the disclosed device is suitable to display the caloric power of the foods to be eaten starting from the weihht amount thereof.

In other words, by supplying the device with data relating to the weight of the single foods comprising a meal, the device, as controlled by the user, will display, on its displaying means, the caloric power of the individual foods and, if required, the caloric power of a full meal.

In this connection, it should be pointed out that the caloric power of each meal can be held in the store of the device for one or more days, thereby providing an useful information about the caloric amount of several subsequent meals, to be eaten according to a set diet.

Thus, the user will be provided with the possibility of accurately knowing, at each time of a day or of a week, the number of the assumed calories, in order to properly control the diet.

While the invention has been disclosed and illustrated with reference to a preferred embodiment thereof, it should be apparent that the disclosed embodiment would be susceptible to several modifications and variations all of which will come within the spirt and scope of the appended Claims.

## Claims

1. A monitoring device for monitoring the caloric contents of diets, characterized in that said device comprises processor means, a first digital keyboard and a second keyboard, the keys of which bear symbols related to single foods, or to a combination of foods, said first and second keyboards controlling said processor means, said processor means including a fixed store suitable to store a unit caloric power of said foods, and display means for displaying said caloric power of said foods as a key of said ditigal keyboard is pressed.

2. A device according to claim 1, wherein said processor means is enclosed in a box-like housing including a plurality of windows, in each of said windows there being engaged a corresponding said key, and a top opening being moreover formed through said box-like housing for holding said display means.

3. A device according to claim 1, wherein said device further comprises a store controlling key, an on switch key and an off switch key.

FIG. 1

FIG. 2

FIG. 3